# EUROPEAN PATENT APPLICATION

(11) **EP 1 298 216 A1**
(43) Date of publication of application: **02.04.2003**
(21) Application number: 01917610.6
(22) Date of filing: 29.03.2001
(51) Int. Cl.: C12P 19/14, C12P 19/02, C12P 1/00

(54) **PROCESS FOR TREATING WITH ENZYME**

(30) Priority: 27.06.2000 JP 2000192105; 18.09.2000 JP 2000281876
(71) Applicant: National Institute of Advanced Industrial Science and Technology, Tokyo 100-88921 (JP)
(72) Inventor: YAMANOBE, Takashi, Ushiku-shi, Ibaraki 300-1235 (JP); OBUCHI, Kaoru, Tsukuba-shi, Ibaraki 305-0032 (JP)
(74) Representative: HOFFMANN EITLE
(86) International application number: JP0102645
(87) International publication number: WO02000912

(57) **Abstract**

A process for enzymatic treatment comprising an enzyme reaction to obtain an enzyme reaction product by allowing an enzyme to act on a substrate, wherein the enzyme reaction is performed under pressure.

## Description

### TECHNICAL FIELD

The present invention relates to a process for enzymatic treatment, and in particular relates to a process for enzymatic treatment which comprises performing an enzyme reaction under pressure.

### BACKGROUND ART

Recently, improvements in environmental pollution levels of ocean, lakes, river, soil and the like, and the related development of environmental conservation energy, such as biomass resources, are now attracting much attention. Chaff, wood, or plants such as sugarcane are used as biomass resources. However, the presence of difficult-to-degrade cellulose and lignin in the above substances hinders their applications. A means to efficiently degrade these substances is necessary.

It is known that glucose is produced by degrading cellulose with an enzyme. Such an enzyme is generally referred to as a cellulase. Cellulase contains 3 types of enzymatic activities: (1) a cellobiohydrolase (avicelase) activity to hydrolyze solid crystal regions of cellulose from the non-reducing end in exo-type mode of action to produce cellobiose, (2) endoglucanase activity (CMCase) to hydrolyze non-crystal regions of cellulose in endo-type mode of action into lower molecules of cellulose and produce various cellooligosaccharides, and (3) β-glucosidase activity to degrade cellobiose or cellooligosaccharide into glucose. By these 3 types of activities, cellulose is degraded into glucose via cellobiose and cellooligosaccharide.

A cellulose degradation reaction using a conventional cellulase is time-consuming because it is performed under atmospheric pressures and temperatures which do not impair enzymatic activity. In addition, under the existing cellulose degradation reaction, it is difficult to allow enzymes to act under stable conditions, so that the yield is inferior and does not reach practical levels.

### DISCLOSURE OF THE INVENTION

In view of environmental conservation and effective utilization of resources as described above, the development of effective treatment and recycling methods for cellulose wastes are anticipated.

Therefore, an object of the present invention is to provide a process for cellulose degradation treatment using cellulase effectively within a short time.

As a result of considerable efforts to solve the above problems, the present inventors have completed the present invention by finding that substrates can be converted efficiently so as to obtain the enzyme reaction products by performing enzyme reactions using various enzymes under pressure, typically, an enzyme reaction for cellulose degradation using cellulase under pressure.

The present invention relates to inventions of the following (1) to (14).
(1) A process for enzymatic treatment comprising an enzyme reaction to obtain an enzyme reaction product by allowing an enzyme to act on a substrate, wherein the enzyme reaction is performed under pressure.
(2) The process of (1) above, which comprises performing an enzyme reaction under a pressure of 0.2 to 1,000 MPa.
(3) The process of (1) or (2) above, which comprises performing an enzyme reaction at temperatures higher than the reaction temperatures employed under atmospheric pressure.
(4) The process of any one of (1) to (3) above, wherein an enzyme is selected from the group consisting of cellulase, glucoamylase, tannase, phytase, protease and chitinase.
(5) The process of any one of (1) to (4) above, wherein the substrate is processed into a powdery, sheet or granular form.
(6) A process for treating with an enzyme, which comprises performing an enzyme reaction under pressure wherein cellulase is allowed to act on cellulose to obtain glucose as an enzyme reaction product.
(7) The process of (6) above, wherein the cellulase is a cellulolytic enzyme system produced by a fungus of the genus *Acremonium.*
(8) The process of (7) above, wherein the fungus of the genus *Acremonium* is *Acremonium* sp. Y-94.
(9) The process of (7) above, wherein an enzyme composition, which is a mixture of the cellulolytic enzyme system produced by a fungus of the genus *Acremonium* and an enzyme of which endoglucanase (CMCase) activity is enhanced under pressure, is used.
(10) The process of (9) above, wherein the enzyme of which CMCase activity is enhanced under pressure is an enzyme produced by a fungus of the genus *Aspergillus.*
(11) The process of (9) above, wherein the mixing ratio of the enzyme produced by a fungus of the genus *Acremonium* and the enzyme of which CMCase activity is enhanced under pressure in the enzyme composition is 7:50 to 50:7 in terms of CMCase activity equivalent.
(12) The process of any one of (6) to (11) above, wherein a pressure of 0.2 to 1,000 MPa is applied to the cellulose and cellulase.
(13) The process of any one of (6) to (12) above, wherein a powdery, sheet-shaped or granular cellulose is used.
(14) The process of any one of (6) to (13) above, wherein a cellulase having CMCase activity equivalent to 0.0005 to 10,000 International Units (IU) is used for 1 g of cellulose.

The present invention will be described in detail as follows. This specification includes part or all of the contents as disclosed in the specification and/or drawings of Japanese Patent Application Nos. 2000-192105 and 2000-281876, which are priority documents of the present application.

In the present invention, an enzyme reaction wherein an enzyme is allowed to act on a substrate to obtain an enzyme reaction product is performed under pressure. Specifically, an enzyme reaction is performed under pressures of 0.2 to 1,000 MPa, preferably, 10 to 500 MPa, and particularly preferably, 100 to 150 MPa. Application of pressure within this range activates enzymes, enhances the enzyme's own stability, and enables reaction at a temperature higher than normal enzyme reaction temperatures. When pressure is higher than the above range, enzyme activity is lost and the reaction efficiency is lowered, and when pressure is lower than the above range, the reaction efficiency is not enhanced to a desired level, and thus both these conditions are unfavorable.

A typical example of an enzyme to be used in the present invention is cellulase, but it is not limited thereto. Examples of such an enzyme that can be similarly used herein include hydrolases, such as glucoamylase, tannase, phytase, protease and chitinase, and also include various oxidoreductases, transferases, isomerases, lyases and synthases. Further, these enzymes may be derived from microorganisms, plants and animals, and commercially available enzymes can also be used.

For example, when cellulase is used, any enzyme which is generally known as a cellulase can be used without any particular limitation. Preferably, the cellulolytic enzyme system produced by a fungus of the genus *Acremonium*, and in particular, the cellulolytic enzyme system produced by *Acremonium* sp. Y-94 can be used.

Further, it is preferable to use an enzyme composition which is a mixture of the above cellulolytic enzyme system produced by a fungus of the genus *Acremonium* and an enzyme of which CMCase activity is enhanced under pressure, because efficiency of cellulose degradation reaction with enzyme is even more enhanced. An example of an enzyme of which CMCase activity is enhanced under pressure is the enzyme produced by a fungus of the genus *Aspergillus.*

When such an enzyme mixture is used, it is exemplified that the mixing ratio of the enzyme produced by a fungus of the genus *Acremonium* and the enzyme of which CMCase activity is enhanced under pressure in the enzyme composition is 7:50 to 50:7 in terms of CMCase activity equivalent.

In the present invention, examples of a substrate on which an enzyme is allowed to act are not specifically limited, so far as each reaction such as hydrolysis, oxidation-reduction, transfer, isomerization, elimination - addition, or synthesis in which the substrate is involved is catalyzed by the enzyme to produce enzyme reaction products. Examples of such a substrate include cellulose, starch, tannic acid, phytic acid, protein and chitin according to each of the above-mentioned enzymes.

When the substrate is cellulose, specific examples that can be used herein include various natural celluloses, such as trees, waste wood, paper or straws.

The shape or the size of this substrate is also not specifically limited. In respect of performing enzyme reaction efficiency, substrates processed into a powdery, sheet or granular form are preferably used.

The mixing ratio of the substrate and the enzyme can be freely selected for each enzyme reaction. For example, in the case of cellulose, enzymes having CMCase activity equivalent to approximately 0.0005 to 10,000 International Units (IU) can be employed for each 1 g of substrate, such as filter paper to be degraded.

Moreover, the enzymatic treatment according to the present invention can be performed under any condition, such as batch, semi-continuous or continuous condition in a chemical reaction chamber which is resistant to normal pressure application.

### BEST MODE FOR CARRYING OUT THE INVENTION

The present invention will be further described in the following examples. These examples are not intended to limit the scope of the invention.

### (Activity measurement)

In the following Examples (comparative examples) 1 to 3, enzymatic activity was expressed in terms of International Units (IU), where 1 IU represents the amount of enzyme capable of degrading cellulose to liberate 1 µmol of reducing sugar per minute under conditions of pH 4.5, 50°C and 0.1 MPa (atmospheric pressure). The measurement of enzymatic activity was conducted by collecting portions of supernatant of reaction solution inactivated by heat treatment after completion of enzyme reaction, diluting it at a fixed ratio, and determining the reducing sugars liberated by the enzyme reaction quantitatively by Somogyi-Nelson method.

### (Example 1 and comparative example 1)

An enzyme reaction for cellulose degradation was performed by applying pressure using water as a pressurizing medium into a stainless steel, pressure-resistant container that held a substrate and an enzyme, namely: onto approximately 150 mg of Whatman No. 1 filter paper (FP) cut into strips, and a cellulase preparation (trade name: Acremozyme) produced by *Acremonium cellulolyticus* having 0.0078 IU of FP saccharifying activity. This container was placed in a thermal bath.

The enzyme and the substrate were added to 2 ml (pH 4.5) of 40 mM Britton-Robinson wide-range buffer (hereinafter, abbreviated as B-R buffer), and then reaction was performed under pressures of 0.1 MPa (atmospheric pressure: Comparative example 1) and 150 MPa (Example 1) at 60°C for 1, 24, 48 and 72 hours, respectively.

Table 1 shows the results. Numerical values for each reaction time in Table 1 represent the amounts of reducing sugar (µg/ml reaction solution) produced, and acceleration ratio (%) values represent the ratios of the amounts of reducing sugar produced at 0.1 MPa to that at 150 MPa.

**Table 1**

| | | | | |
|---|---|---|---|---|
| Reaction time (Hr) | 1 | 24 | 48 | 72 |
| Example 1 | 119 | 1497 | 1986 | 2335 |
| Comparative example 1 | 50 | 210 | 277 | 299 |
| Accerelation ratio (%) | 238 | 713 | 717 | 780 |

### (Example 2 and comparative example 2)

An enzyme reaction for cellulose degradation was performed in a manner similar to Example 1, except that reaction temperature was 65°C. Table 2 shows the results.

**Table 2**

| | | | | |
|---|---|---|---|---|
| Reaction time (Hr) | 1 | 24 | 48 | 72 |
| Example 2 | 218 | 1744 | 2640 | 2876 |
| Comparative example 2 | 38.9 | 88.5 | 121.6 | 141.4 |
| Accerelation ratio (%) | 560 | 1971 | 2171 | 2034 |

### (Example 3 and comparative example 3)

An enzyme reaction for cellulose degradation was performed by applying pressure using water as a pressurizing medium into a stainless steel, pressure-resistant container that held a substrate and a mixed enzyme, namely: approximately 150 mg of Whatman No. 1 filter paper (FP) cut into strips, and a mixture of a cellulase preparation (trade name: Acremozyme) produced by *Acremonium cellulolyticus* having 0.0078 IU of FP saccharifying activity and a cellulase preparation (trade name: Cellsoft Ultra) produced by the genus *Aspergillus* having 0.0188 IU of CMCase activity. This container was placed in a thermal bath.

The enzyme and the substrate were added to a 2 ml of 40 mM B-R buffer (pH 4.5), and then reaction was performed under pressures of 0.1 MPa (atmospheric pressure: Comparative example 3) and 150 MPa (Example 3) at 65°C for 1, 8, 24 and 48 hours, respectively.

Table 3 shows the results.

**Table 3**

| | | | | |
|---|---|---|---|---|
| Reaction time (Hr) | 1 | 8 | 24 | 48 |
| Example 3 | 307 | 1521 | 2300 | 5443 |
| Comparative example 3 | 72 | 228 | 271 | 365 |
| Accerelation ratio (%) | 426 | 667 | 849 | 1491 |

### (Example 4 and comparative example 4)

As an enzyme, 1 mg of glucoamylase produced by *Rhizopus niveus* (SEIKAGAKU CORPORATION) was dissolved in a 1 ml of 40 mM B-R buffer (pH 4.5), thereby preparing an enzyme stock solution. As a substrate, soluble starch was added to B-R buffer so as to give a concentration of 5% (w/v) and dissolved by heating, thereby obtaining a substrate solution.

120 µl of an enzyme solution prepared by diluting the above enzyme stock solution by 128-fold was added to and mixed with 11.88 ml of the above substrate solution. Then reaction was performed for 24 hours at pH 4.5 under pressures of 0.1 MPa (atmospheric pressure: Comparative example 4) and 150 MPa (Example 4) at 45°C, 55°C and 65°C, respectively. After reaction, the enzyme was inactivated by heating in boiling water for 10 minutes. The supernatant of reaction solution was diluted by an appropriate ratio. Then, the amount of reducing sugar (reaction product) produced in the supernatant was calculated by subjecting 1 ml of the diluted solution to the Somogyi-Nelson method.

Table 4 shows the results. Numerical values for each reaction temperature in Table 4 represent the amounts of reducing sugar (µg/ml reaction solution) produced, and acceleration ratio (%) values represent the ratios of the amounts of reducing sugar produced at 0.1 MPa to that at 150 MPa.

**Table 4**

| | | | |
|---|---|---|---|
| Reaction temperature (°C) | 45 | 55 | 65 |
| Example 4 | 712 | 1252 | 1738 |
| Comparative example 4 | 841 | 686 | 441 |
| Accerelation ratio (%) | 84.7 | 182.5 | 394.1 |

### (Example 5 and comparative example 5)

As an enzyme, 5 mg of tannase produced by *Aspergillus oryzae* (Wako Pure Chemical Industries, Ltd.) was dissolved in 5 ml of 40 mM B-R buffer (pH 5.5), thereby preparing an enzyme stock solution. As a substrate, tannic acid was dissolved in distilled water so as to give a concentration of 2 % (w/v).

8.9 ml of B-R buffer, 1 ml of 2% tannic acid aqueous solution and 100 µl of an enzyme solution prepared by diluting the above enzyme stock solution by 80-fold were mixed. Reaction was then performed for 60 hours at pH 5.5 under pressures of 0.1 MPa (atmospheric pressure: Comparative example 5) and 150 MPa (Example 5) at 30°C and 55°C, respectively. After reaction, the enzyme was inactivated by heating in boiling water for 10 minutes. 4 ml of 80% ethanol was added to 1 ml of the reaction solution, and then the amount of decrease in absorbances at 310 nm wavelength was measured. The amount of the reaction product was indirectly calculated from the amount of the residual substrate after degradation by the enzyme reaction.

Table 5 shows the results. Numerical values for each reaction temperature in Table 5 represent absorbances at 310 nm wavelength, and acceleration ratio (%) values represent the ratios of absorbances at 0.1 MPa to that at 150 MPa.

**Table 5**

| | | |
|---|---|---|
| Reaction temperature (°C) | 30 | 55 |
| Example 5 | 0.2851 | 0.8865 |
| Comparative example 5 | 0.2897 | 0.6812 |
| Accerelation ratio (%) | 98.4 | 130 |

### (Example 6 and comparative example 6)

As an enzyme, 20 mg of the wheat-derived phytase (Sigma Chemicals) was dissolved in 2 ml of 50 mM acetate buffer (pH 5.15), thereby preparing an enzyme stock solution. As a substrate, Inositol hexaphosphoric acid dodecasodium salt derived from rice was added and dissolved in the above buffer so as to give a concentration of 2% (w/v), thereby obtaining a substrate solution.

100 µl of an enzyme solution prepared by diluting the above enzyme stock solution by 8-fold was added to and mixed with 9.9 ml of the above substrate solution. Reaction was then performed for 24 hours at pH 5.15 under pressure of 0.1 MPa (atmospheric pressure: Comparative example 6) and 150 MPa (Example 6) at 55°C and 65°C, respectively. After reaction, the enzyme was inactivated by heating in boiling water for 10 minutes. Quantitative determination of inorganic phosphorus liberated by the enzyme reaction was performed according to the Fiske-Subbarrow method. Specifically, to 1 ml of the above reaction solution, 6.6 ml of distilled water, 1 ml of 2.5% ammonium molybdate, 1 ml of 3N sulfuric acid, and 0.4 ml of amino naphthol sulfonate (195 ml of 15% sodium acid sulfite and 5 ml of 20% sodium sulfite 7-hydrate were added to and dissolved in 0.5 g of 1,2,4 amino naphthol sulfonate) were added. After mixing, the solution was allowed to stand for 30 minutes, and then absorbance at 750 nm wavelength was measured, so that inorganic phosphorus level was calculated.

Table 6 shows the results. Numerical values for each reaction temperature in Table 6 represent inorganic phosphorus levels (µg/ml reaction solution), and acceleration ratio (%) values represent the ratios of inorganic phosphorus levels at 0.1 MPa to these at 150 MPa.

**Table 6**

| | | |
|---|---|---|
| Reaction temperature (°C) | 55 | 65 |
| Example 6 | 9.2 | 41.6 |
| Comparative example 6 | 16.0 | 25.3 |
| Accerelation ratio (%) | 57.5 | 164.4 |

### (Example 7 and comparative example 7)

As an enzyme, 10 mg of the *Bacillus subtilis*-derived a protease preparation "Protease N "Amano" (AMANO PHARMACEUTICAL CO., LTD) was dissolved in 1 ml of 40 mM B-R buffer (pH 7.0), thereby preparing an enzyme stock solution. As a substrate, Nutrose [sodium caseinate (Wako Pure Chemical Industries Ltd.)] was suspended in 40 mM B-R buffer (pH 7.0) so as to give a concentration of 1% (w/v), and then dissolved in hot water for 15 minutes, thereby obtaining a substrate solution.

100 µl of an enzyme solution prepared by diluting the above enzyme stock solution by 700-fold was added to and mixed with 9.9 ml of the above substrate solution. Reaction was then performed for 24 hours at pH 7.0 under pressures of 0.1 MPa (atmospheric pressure: Comparative example 7) and 150 MPa (Example 7) at 55°C and 70°C, respectively. After reaction, the enzyme was inactivated by adding 3 ml of 5% trichloroacetic acid (TCA). Absorbance at 280 nm wavelength was measured for the reaction supernatant solution.

Table 7 shows the results. Numerical values for each reaction temperature in Table 7 represent absorbances at 280 nm wavelength, and acceleration ratio (%) values represent the ratios of absorbances at 0.1 MPa to that at 150 MPa.

**Table 7**

| | | |
|---|---|---|
| Reaction temperature (°C) | 55 | 70 |
| Example 7 | 0.1535 | 0.1795 |
| Comparative example 7 | 0.1106 | 0.0800 |
| Accerelation ratio (%) | 138.8 | 224.4 |

### (Example 8 and comparative example 8)

As an enzyme, 5 mg of the *Streptomyces griseus*-derived chitinase (Sigma Chemical) was dissolved in 36 ml of 40 mM B-R buffer (pH 6.0), thereby preparing an enzyme solution. As a substrate, 20 mg of chitin was measured into a 2 ml cuvette.

2 ml of the above enzyme solution was added to the 2 ml cuvette and mixed with the 20 mg of chitin contained therein. Then reaction was performed for 24 hours at pH 6.0 under pressures of 0.1 MPa (atmospheric pressure: Comparative example 8) and 150 MPa (Example 8) at 30°C and 60°C, respectively. After reaction, the enzyme was inactivated by heating in boiling water for 10 minutes. The supernatant of reaction solution was appropriately diluted, and then quantitatively determined using a TOC analyzer (Total Organic Carbon analyzer).

Table 8 shows the results. Numerical values for each reaction temperature in Table 8 represent TOC (TOC mg/ml reaction solution), and acceleration ratio (%) values represent the ratios of TOC at 0.1 MPa to TOC at 150 MPa.

**Table 8**

| | | |
|---|---|---|
| Reaction temperature (°C) | 30 | 60 |
| Example 8 | 0.13 | 0.90 |
| Comparative example 8 | 0.24 | 0.80 |
| Accerelation ratio (%) | 54.1 | 112.5 |

All publications, patents and patent applications cited herein are incorporated herein by reference in their entirety.

### Industrial Applicability

According to the present invention, there is provided a process for enzymatic treatment which can efficiently perform an enzyme reaction within a short time. In the process of the present invention, pressure application activates the enzyme and improves the enzyme's own stability, so as to make it possible for reactions to take place at temperatures higher than normal enzyme reaction temperatures. Enzymatic process for cellulose degradation performed by the process of the present invention enables effective treatment of large quantities of generated cellulose waste. Thus, the process of the present invention is very useful in terms of both environmental conservation and effective utilization of resources.

## Claims

1. A process for enzymatic treatment comprising an enzyme reaction to obtain an enzyme reaction product by allowing an enzyme to act on a substrate, wherein the enzyme reaction is performed under pressure.

2. The process of claim 1, which comprises performing an enzyme reaction under a pressure of 0.2 to 1,000 MPa.

3. The process of claim 1 or 2, which comprises performing an enzyme reaction at temperatures higher than the reaction temperatures employed under atmospheric pressure.

4. The process of any one of claims 1 to 3, wherein an enzyme is selected from the group consisting of cellulase, glucoamylase, tannase, phytase, protease and chitinase.

5. The process of any one of claims 1 to 4, wherein the substrate is processed into a powdery, sheet or granular form.

6. A process for treating with an enzyme, which comprises performing an enzyme reaction under pressure wherein cellulase is allowed to act on cellulose to obtain glucose as an enzyme reaction product.

7. The process of claim 6, wherein the cellulase is a cellulolytic enzyme system produced by a fungus of the genus *Acremonium.*

8. The process of claim 7, wherein the fungus of the genus *Acremonium* is *Acremonium* sp. Y-94.

9. The process of claim 7, wherein an enzyme composition which is a mixture of the cellulolytic enzyme system produced by a fungus of the genus *Acremonium* and an enzyme of which endoglucanase (CMCase) activity is enhanced under pressure is used.

10. The process of claim 9, wherein the enzyme of which CMCase activity is enhanced under pressure is an enzyme produced by a fungus of the genus *Aspergillus.*

11. The process of claim 9, wherein the mixing ratio of the enzyme produced by a fungus of the genus *Acremonium* and the enzyme of which CMCase activity is enhanced under pressure in the enzyme composition is 7:50 to 50:7 in terms of CMCase activity equivalent.

12. The process of any one of claims 6 to 11, wherein a pressure of 0.2 to 1,000 MPa is applied to the cellulose and cellulase.

13. The process of any one of claims 6 to 12, wherein a powdery, sheet-shaped or granular cellulose is used.

14. The process of any one of claims 6 to 13, wherein a cellulase having CMCase activity equivalent to 0.0005 to 10,000 International Units (IU) is used for 1 g of cellulose.
